# EUROPEAN PATENT APPLICATION

(11) **EP 1 852 416 A1**
(43) Date of publication of application: **07.11.2007**
(21) Application number: 06425304.0
(22) Date of filing: 04.05.2006
(51) Int. Cl.: C07C 231/20, C07C 237/06

(54) **Enantiomeric resolution of a carnitinamide salt by preferential crystallization**

(71) Applicant: UNIVERSITA' DEGLI STUDI DI MILANO, 20122 Milano (IT); Piccolo, Oreste, 23896 Sirtori (IT)
(72) Inventor: Pallavicini, Marco, 20031 Cesano Maderno (MI) (IT); Valoti, Ermanno, 24044 Dalmine (BG) (IT); Bolchi, Cristiano, 20010 Bareggio (MI) (IT); Fumagalli, Laura, 20050 Triuggio (MI) (IT); Piccolo, Oreste, 23896 Sirtori (LC) (IT)
(74) Representative: Minoja, Fabrizio

(57) **Abstract**

A process for the resolution of (D,L)-carnitinamide **(D,L-I)** chloride in its enantiomers **(D-I)** and **(L-I),** characterized in that a supersaturated solution or suspension of **(D,L-I)** in a suitable solvent or mixture of solvents, optionally added with a surfactant, is used. A series of alternate preferential crystallizations is carried out after adding minimum amount of seeds of one of the two enantiomers, **(D-I)** or **(L-I),** that is to be precipitated, and subsequently restoring the supersaturation conditions by adding an appropriate amount of **(D,L-I)** racemate.

## Description

Object of the present invention is a process for the resolution of D, L-Carnitinamide chloride **(DL-I)** in its enantiomers by alternate preferential crystallization of the single enantiomer **(L-I)** and **(D-I)** from supersaturated solutions of the racemate, containing a slight excess of one or the other of the two enantiomers, by seeding small amounts of crystalline seeds of the enantiomer to be precipitated.

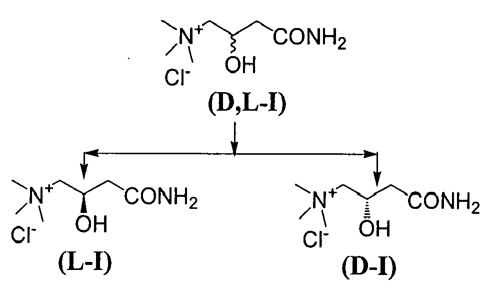

The enantiomer **(L-I)** is a useful intermediate in the preparation of L-carnitine and its derivatives. Both enantiomers **(L-I)** and **(D-I)** are also precursors, respectively, of (R)- and (S)-3-hydroxy-γ-butyrolactone, important and versatile chiral synthons.

### Field of the invention

The present invention relates to a process for the resolution of Carnitinamide chloride racemate **(D,L-I)** in its corresponding enantiomers.

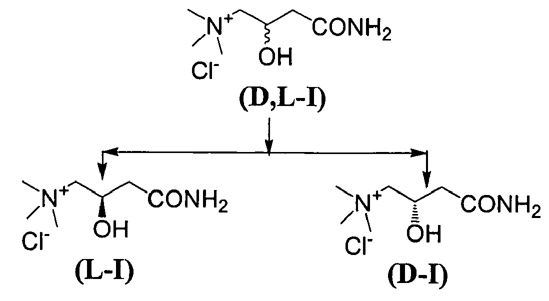

The enantiomer **(L-I)** is an immediate precursor of L-carnitine (Vitamin Bₜ) **(II),** that is presently prepared, in one of the main industrial processes, by hydrolysis of the amide as disclosed in US 4254053 and US 4521285.

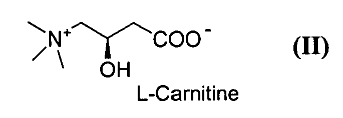

The importance of compound **(II)** is well known. This hydroxybutyrobetaine is implied in numerous metabolic processes (oxidation of fatty acids, synergy in the metabolism of carbohydrates, increment of the speed in the oxidative phosphorylation) and has many therapeutic (cardiomyopathies, cardiovascular ischaemias, deficiencies of carnitine of various origin) and nutraceutical uses (as an adjuvant in sportsmen and elderly people, in therapies for weight control, muscular weakness and chronic fatigue, as an adjuvant in increasing memory and concentration abilities, as a cattle nutritional supplement). Its world-wide market, that is estimated to-day equal to multi hundreds of tons, is destined to grow, both in the pharmaceutical and nutraceutical fields.

From some decades the methods of preparation of **(II)** (only this L-enantiomer is used) are object of intense searches, finalized to select and optimize the most convenient stereotechnology for its obtaining. The number of patents and publications is remarkable as a further proof of the industrial and commercial interest towards this product. These works can be subdivided in two main categories: one based on processes for the resolution of carnitine and its precursors starting from the corresponding racemic mixtures, optionally coupled to procedures for the re-use of the undesired antipode with the aim to diminish the amount of wastes (US 6395923); the other based on processes finalized to obtain only the desired enantiomer.

To the best of our knowledge, the main procedures currently used on industrial scale are: a) the resolution of the salt of the D,L carnitinamide with D-camphoric acid (US 4254053 and loc. cit.) and following hydrolysis to (II); b) the stereospecific hydration of crotonobetaine by means of a biological method (US 4708936). The former method is reliable, it uses an economic starting material, **(D,L-I),** and has a good yield. Its disadvantages are low productivity, high quantities of wastes, number of total steps for the transformation of **(D,L-I)** in the corresponding hydroxy derivative (salification with D-camphoric acid, selective crystallization, recovery of the resolving agent, isolation of the L-carnitinamide chloride or sulfate, purification); the need to recover the resolving agent, D-camphoric acid, which is expensive and not always easily available, and to re-use the undesired enantiomer. Also the latter procedure is reliable and uses the cheap γ-butyrolactone, as starting material. It requires the oxidation of 4-butyrobetaine or 4-dehydrobutyrobetaine. Its productivity is good but it involves drawbacks due intensive laborious work-up procedures, high quantities of wastes and need to use a modified microorganism to prevent undesired oxidation to β-ketoacid.

In addition to these two methods, other procedures have been studied and developed but they have found up today only limited or no industrial application, because of intrinsic disadvantages; the most relevant ones may be mentioned: use of chiral synthons [such as (S)-epichlorohydrin or its analogoues, used in JP3287567 and US4814506, or optically active (S)-3-hydroxybutyrolactone reported in US 6342034)]; asymmetric reduction by means of enantiomerically pure homogenous catalysts (US6566552 and loc. cit.); enzymatic reduction; enzymatic water addition to 4-dehydrobutyrobetaine; resolution of carnitine by diastereoselective crystallization of diastereomeric salts. Among these last methods, in particular, it is important to underline some procedures for the resolution of racemic carnitine nitrile (D, L-III), a synthetic precursor of carnitinamide, by preferential crystallization of the single enantiomers of some its salts from supersaturated solutions of the racemic compound, containing a slight excess of the enantiomer subsequently to crystallize.

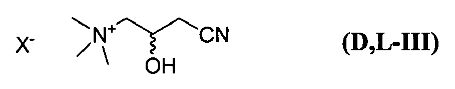

This type of resolution, usually called resolution by "entrainment", is possible only when the product crystallizes as a conglomerate and requires a systematic search to select the favourable conditions, if any, as it will be better described later, in order to efficiently obtain both enantiomers in good chemical yields and e.e. When possible, , this procedure is convenient as it requires no resolving agents, with the exclusion of slight amounts of pure enantiomers [see J. Jacques, A. Collet e S.H. Wilen "Enantiomers, racemates and resolutions", pag. 223-241, Wiley (1981); A. Collet, Enantiomer, 4,157 (1999)].

However, the resolution of **(D,L-III)** (GB1198974) shows a poor efficiency. On the basis of the limits of such a procedure, other resolution procedures by entrainment of some **(D,L-III)** salts were described: the corresponding mono oxalate and perchlorate in alcoholic or hydroalcoholic solvents and the chloride, using a mixture of methoxyethanol and water as the solvent (FR 2536391). According to the inventors, the resolutions carried out under these conditions provide typical efficiencies of entrainment methodology and considerably better results than those achieved according to GB 1198974.

In this context, it should however be stressed that:
- In spite of the convenience and industrial applicability of resolutions by entrainment, the procedures described in FR 2536391 for the salts of **(D,L-III)** have not found application on industrial scale.
- In order to acceptably resolve **(D,L-III),** it is necessary first to transform the most accessible chloride (**D,L-III;** X⁻=Cl⁻) into the corresponding hydroxide and then in another salt, such as mono oxalate (in any case the preferred one) or perchlorate; alternatively, when using the chloride, such a toxic crystallization solvent a methoxyethanol should be used and the resulting efficiency is however lower than that achieved by oxalate.
- Hydrolysis conditions to transform optically active carnitina nitrile into L-carnitine, through the formation of intermediate carnitinamide, can induce partial phenomena of racemisation, in particular in the step nitrile→ amide.

Surprisingly, although the conglomerate nature of carnitinamide chloride **(D,L-I)** has been known since the eighties [J.Prakt.Chem., Bd. 322, 785-792 (1980)], to the best of our knowledge, the resolution of this compound by preferential crystallization has never been described. Even more surprisingly, according to the present invention, preferential crystallization of **(D,L-I)** has successfully been applied, thus developing a simple, economic procedure, which is suitable for large scale production. Both enantiomers are recovered in good chemical yield and satisfactory optical purity, that, according to known methodologies (US 4254053, US 4521285, US 5714619, US 6395923), may be used for the industrial production of L-carnitine, its derivatives and several chiral synthons.

### Detailed description of the invention

Object of the present invention is a process for the resolution of (D,L)-carnitinamide **(D,L-I)** chloride in its enantiomers **(D-I)** and **(L-I),** characterized in that a supersaturated solution or suspension of **(D,L-I)** in a suitable solvent or mixture of solvents, optionally added with a surfactant, is used. A series of alternate preferential crystallizations is carried out after adding minimum amount of seeds of one of the two enantiomers, **(D-I)** or **(L-I),** that is to be precipitated, and subsequently restoring the supersaturation conditions by adding an appropriate amount of **(D,L-I)** racemate. In more detail, said process comprises the following steps:
a) Preparation of a supersaturated solution or emulsion of a racemate of formula **(D,L-I)** in a suitable solvent or mixture of solvents, optionally adding surfactants, preferably non ionic surfactants;
b) Seeding with a minimum amount of crystals of one of the two enantiomers;
c) Collection of the precipitate, preferably by filtration;
d) Dilution of the mother liquors with the same solvent or mixture of solvents as used in step a) to the starting volume and addition of racemate in such an amount as to restore the starting supersaturation conditions;
e) Seeding with the minimum amount of seeds of the other enantiomer (the antipode of the enantiomer used in step b));
f) Collection of the precipitate, preferably by filtration;
g) Dilution of the mother liquors with the same solvent or mixture of solvents as used in step a) to the starting volume and addition of the racemate in such an amount as to restore the starting supersaturation conditions;
h) Repetition of steps b)-g) for an indefinite number of times.

If desired, the single pure enantiomers may be collected and the product recrystallized. The supersaturated solution or emulsion of steps a), d) and g) is prepared by heating to boiling temperature and the crystallization is induced upon cooling the mixture to a suitable temperature. The starting solution or emulsion may also contain an excess of the enantiomer subsequently precipitated in step c), ranging from 0 to 10%, preferably from 3 to 5% on the weight of the racemate). If this enantiomer is present, steps b) and i) can also be omitted.

The minimum amount of seed of pure enantiomer, added to initiate the crystallization, is preferably comprised between 0.05% and 0.2% on the overall amount of compound **(D,L-I)** in solution.

The solvent is preferably a C1-C4 alcohol, optionally mixed with water, more preferably methanol or ethanol or their mixtures with water.

According to a prefereed embodiment of the invention, **(D,L-I)** racemate is resolved using methanol as the solvent, because the solubility of **(D,L-I)** in this solvent is twice that of the single enantiomers.

If present, the surfactant is preferably a non ionic surfactant.

The concentration of the supersaturated solution is in the range of 10-30%, preferably 12-20%.

The crystallization is carried out at 20-40°C, preferably at 25-35°C.

The present invention also relates to a process to obtain L-carnitine and (S)- or (R)-3-hydroxy-γ-butyrolactone starting from **(D-I)** and **(L-I)** obtained by the resolution method described above.

In the Table, the results of chemical yield and optical purity achieved by ten consecutive preferential crystallizations from methanol are reported, starting from 4 g of **(D,L-I),** initially enriched with 200 mg of pure **(L-I).**

The crystallized fractions of **(L-I)** and **(D-I)** were respectively combined and once recrystallized from methanol, thereby enhancing the optical purities from 75.5% to 100% and from 74.3% to 98.1%, respectively. The advantages of the present invention may be so summarized:
a) The only starting material necessary, namely racemic carntinamide chloride, is a commercially available, cheap product; the crystallization solvents are economic, widely used and easily recovered;
b) There is no need for a chiral auxiliary (as disclosed, for instance, in US 4254053), with the exclusion of the possible, minimum starting enrichment in one of the two enantiomers and the negligible amounts of enantiopure seeds used to trigger crystallization; therefore, any problems connected with the recovery and recycle of the auxiliary are avoided and no further reactors are required;
c) The procedure is very simple, as no specific workup is required, but it only consists in crystallizations and filtrations; furthermore, once experimental conditions are standardized, the process is highly reproducible as far as chemical and optical yields (i.e. amount of enantiomerically pure product in the precipitate) are concerned;
d) The resolution efficiency (chemical yield x optical purity or enantiomeric excess) is absolutely good (approx. 20% recovery of total enantiomer at each crystallization) and excellent in comparison with the efficiencies typical of resolutions by entrainment;
e) The volumetric productivity is reasonable [concentration in supersaturated solution (w/w) ranging between 10 and 30%, preferably between 12 and 20%];
f) Crystallization cycles may be repeated many and many times, since no impurities due to partial degradation of the substrate are observed in the mother liquors;
g) There is no need to transform the chloride into other salts, such as mono oxalate or perchlorate, to obtain successful resolution, which is instead required in the case of carnitine nitrile resolution by entrainment;
h) No specific solvent systems are required, as is the case with carnitina nitrile chloride, which is resolved (in comparatively poor yields) by preferential crystallization from toxic methoxyethanol and water;
i) The quality of L-carnitine prepared according to known procedures, from **(L-I)** obtained according to the invention fulfils the required market specifications;
j) The quality of L-carnitine and of other chiral synthons, such as (R)- and (S)-3-hydroxy-γ-butyrolactone, prepared according to known procedures from **(L-I)** and **(D-I)** obtained according to the invention fulfils the required market specifications.

For these reasons, this procedure is suitable for the application on a large scale.

The following example illustrates the invention more in detail.

### Resolution of racemic carnitinamide chloride by crystallization in methanol.

A stirred mixture of **(D,L-I)** (4 g), **(L-I)** (0.2 g) and methanol (30 g) was heated to boiling temperature to obtaine a clear solution. Afterwards, the solution was at 32±5°C and added with seeds of **(L-I)** (approx. 5 mg) while stirring. In 10-15 min at the same temperature, a colourless precipitate formed and was isolated by filtration and immediately dried without being washed on the filter, thereby obtaining:
- Mother liquors (filtrate): α_{D}³⁰ = +0.086
- Precipitate of **(L-I)** (420 mg): [α]_{D}²⁰ = -19.50 (c 1, MeOH) (79.6% optical purity resulting from the percent ratio of the observed specific rotation to the specific rotation of -24.49).

Mother liquors were diluted with methanol to the starting amount (30 g) and added with **(D,L-I)** (420 mg). The stirred mixture was heated to boiling temperature to obtain a clear solution which was cooled while stirring. Seeds of **(D-I)** (approx. 5 mg) were added at 32±5°C. After that, the procedure described above was repeated, thereby obtaining:
- Mother liquors (filtrate): α_{D}³⁰ = -0.074
- Precipitate of **(D-I)** (410 mg): [α]_{D}²⁰ = +17.92 (c 1, MeOH) (73.2% optical purity resulting from the percent ratio of the observed specific rotation to the specific rotation of +24.49.

Then a further four preferential crystallizations of the levo enantiomer were carried out (crystallizations III, V, VII and IX in Table) alternated with four preferential crystallizations of the dextro enantiomer (crystallizations IV, VI, VIII and X in Table), each time adding an amount of racemate equivalent that amountof the optically active precipitate, isolated in the previous cycle. From crystallization IV to crystallization X, the amount of methanol was increased from 30 g to 32 g and the crystallization times from about 15 min in the first three crystallizations to about 60 min in the subsequent seven resolutions. In each crystallization experiment, the moment when it was convenient to filtrate was decided on the basis of the reached degree of turbidity of the methanolic mixture.

After ten crystallization cycles, 2.461 g of **(L-I)** (optical purity = 75.5%) and 2.605 g of **(D-I)** (optical purity = 74.3%) were recovered. By recrystallization of these two amounts from methanol, 1.800 g of **(L-I)** ([α]_{D}²⁰ = -25.0 (c 1, MeOH); [α]_{D}²⁰ = -17.13 (c 2, water); 100% o.p.) and 1.874 g of (**D-I**) ([α]_{D}²⁰ =+24.02 (c 1, MeOH); [α]_{D}²⁰ = +16.70 (c 2, water); 98% o.p.) were respectively obtained.

The results of the whole procedure are reported in the Table.

The mother liquors from the last crystallization showed -0.131 optical activity and, after concentration, afforded 3.510 g of carnitinamide chloride enriched in the levo enantiomer ([α]_{D}²⁰ = -2.36 (c 1, MeOH); 9.6% optical purity). These values are consistent with those of the mixture initially subjected to resolution, i.e. 4.2 g and 4.8% optical purity (4 g of racemate + 200 mg of levo enantiomer).NMR analysis (300 MHz) of the solid resulting from the final concentration of mother liquors did not show any degradation of the substrate during the repeated crystallizations.

**Table**

| Resolution of **(D,L-I)** by ten preferential crystallizations from methanol (30 g of methanol in I-III crystallizations and 32 g of methanol in IV-X crystallizations) | | | | | | |
|---|---|---|---|---|---|---|
| cryst. | precip. time | added carnitinamide chloride, *mg* | | recovery of resolved carnitinamide chloride, mg | | α_{D} of the mother liquors |
| | | racemate | (-) | (-) | (+) | |
| I | 12' | 4000 | 200 | 420 (o.p. 79.6%) | | +0.086 |
| II | 14' | 420 | | | 410 (o.p. 73.2%) | -0.074 |
| II | 15' | 410 | | 400 (o.p. 63.8%) | | +0.090 |
| IV | 46' | 400 | | | 560 (o.p. 64.8%) | -0.125 |
| V | 31' | S60 | | 530 (o.p. 75.1%) | | +0.100 |
| VI | 75' | 530 | | | 605 (o.p. 65.3%) | -0.108 |
| VII | 42' | 605 | | 570 (o.p. 76.3%) | | +0.120 |
| VIII | 80' | 570 | | | 520 (o.p. 91.2%) | -0.150 |
| IX | 56' | 520 | | 541 (o.p. 80.3%) | | +0.102 |
| X | 105' | 541 | | | 510 (o.p. 79.2%) | -0.131 |
| Total, mg | | 8556 | 200 | | | |
| Residue, mg | | 3510 | | 2461* | 2605* | |
| | | (L enriched; | | (o.p. 75.5%) | (o.p. 74.3%) | |
| | | o.p. 9.6%) | | | | |
| Lost, *mg* | | 180 | | | | |
| *1800 mg (o.p. 100%) after one recrystallization from methanol | | | | | | |
| ** 1874 mg (o.p. 98.1%) after one recrystallization from methanol | | | | | | |

## Claims

1. A process for the resolution of (D,L)-carnitinamide **(D,L-I)** chloride in its enantiomers **(D-I)** and **(L-I), characterized in that** a supersaturated solution or suspension of **(D,L-I)** in a suitable solvent or mixture of solvents, optionally added with a surfactant, is used. A series of alternate preferential crystallizations is carried out after adding minimum amount of seeds of one of the two enantiomers, (**D-I**) or (**L-I**), that is to be precipitated, and subsequently restoring the supersaturation conditions by adding an appropriate amount of **(D,L-I)** racemate.

2. A process as claimed in claim 1, wherein the solvent is a C1-C4 alcohol, optionally mixed with water.

3. A process as claimed in claim 2, wherein the solvent is methanol or ethanol, optionally in admixture with water.

4. A process as claimed in claim 3, wherein the solvent is methanol.

5. A process as claimed in claim 1, wherein the surfactant is a non ionic surfactant.

6. A process as claimed in claims 1-3, wherein the concentration of the supersaturated solution is in the range of 10-30%.

7. A process as claimed in claims 1-4, wherein crystallization is carried out at 20-40°C.

8. A process as claimed in claims 1-5, wherein the minimum amount of seed of the pure enantiomer is comprised between 0.05% and 0.2% on the amount of compound **(D,L-I)** in solution.

9. A process as claimed in claims 1-6, wherein starting supersaturated **(D,L-I)** solution also contain the enantiomer subsequently precipitated in amounts from 0 to 10%.

10. A process for the preparation of L-carnitine and (S) or (R) 3-hydroxy-γ-butyrolactone, starting from **(D-I)** and **(L-I)** obtained as claimed in claims 1-9.
